# Europäisches Patentamt
# European Patent Office
# Office européen des brevets

(11) Publication number: **0 106 439**
**B1**

(12) **EUROPEAN PATENT SPECIFICATION**

(45) Date of publication of patent specification: **11.11.87**

(51) Int. Cl.⁴: **A 61 F 13/00, A 61 L 15/01**

(21) Application number: **83304525.5**

(22) Date of filing: **05.08.83**

(54) **Wound dressing and its manufacture.**

(30) Priority: **12.08.82 GB 8223253**

(43) Date of publication of application:
**25.04.84 Bulletin 84/17**

(45) Publication of the grant of the patent:
**11.11.87 Bulletin 87/46**

(84) Designated Contracting States:
**BE CH DE FR GB IT LI NL SE**

(56) References cited:
**EP-A-0 050 514**
**AT-B- 145 334**
**DE-A-1 642 125**
**DE-B-1 254 294**
**DE-U-7 312 783**
**DE-U-7 703 897**
**GB-A-2 074 029**
**US-A-2 877 765**
**US-A-3 648 692**
**US-A-3 927 669**

(73) Proprietor: **Smith and Nephew Associated Companies p.l.c.**
**2, Temple Place Victoria Embankment**
**London WC2R 3BP (GB)**

(72) Inventor: **Lang, Stephen Michael**
**4 New Cottages Wicken Bonhunt**
**Nr. Saffron Walden Essex CB11 3UQ (GB)**
Inventor: **Webster, David Fitzgerald**
**10 Portland Road**
**Bishops Stortford Hertfordshire (GB)**

(74) Representative: **Cole, William Gwyn**
**Corporate Patents Department Smith and Nephew Research Limited Gilston Park Harlow Essex CM20 2RQ (GB)**

## Description

The present invention relates to an absorptive wound dressing suitable for use on burns or other wounds which dressing has a reduced tendency to adhere to the wound. The present invention also relates to the manufacture and use of such dressings.

Burns and other related wounds such as donor sites and the like present a serious problem in that they tend to produce large amounts of exudate which can cause conventional dressings to become saturated or to stick to the wound or even to become infected. One method of covering such wounds has been to cover the wound with a material into which new epithelial or fibroblast growth can penetrate. Dressings of this kind are disclosed in U.S. Patents Nos. 3526224, 3648692 and 3949742.

However such dressings can be extremely painful to remove and often require surgical excision. A fundamentally different approach requiring a fundamentally different type of dressing is to employ materials that are designed to reduce the propensity to adhere to the wound. Dressings of this kind are disclosed in British Patent No. 439085, French Patent No. 947609, United States Patents Nos. 3543750, 2923298 and British Patent No. 778813 which later patents cover successfully used materials such as Melolin ("Melolin" is a registered Trade Mark of T. J. Smith and Nephew Limited, Welwyn Garden City, Herts., U.K.). One more recent attempt at non-adherent dressings is United States Patent No. 3709221 which discloses a dressing having an outer microporous, liquid repellent fibrous layer, an inner macroporous fibrous layer and an absorbent intermediate layer which was also envisaged as normally being fibrous. In order to reduce the tendency of this material to adhere to the wound the inner layer had to be treated with an agent to render it non-wetted by body liquid. It is now realised that it would be desirable to provide a dressing in which the wound facing layer did not require special treatment. As it will become apparent hereinafter it has now been discovered that by avoiding fibrous materials it is possible to produce a dressing with reduced tendency to adhere to wounds without the need for special treatments. An attempt at producing an absorbent dressing is described in U.S. Patent No. 3888248 which describes a dressing fabricated from at least four sheet materials. The wound facing part of the dressing apparently consists of a grid or scrim coated with polyethylene in such a manner that the polyethylene surrounds the filaments of the grid and collects any loose thread or particle that may be present in the core material. It is now realised that it is desirable to avoid the use of wound facing layers that can allow such penetration of the central layer to the wound surface. It has also been realised that it would be desirable to provide a material that was highly conformable to the wound so that it is possible to minimise the quantity of exudate between the wound surface and the dressing. U.S. Patents Nos. 3709221 and 3888248 disclose materials which are bonded along their edges which may reflect a desired to improve conformability. The dressing of the present invention allows for bonding over the whole of the operative area while retaining flexibility.

United States Patent No. 3927667 discloses a low adherency wound dressing which comprises a wound facing layer formed from a film having a plurality of openings, an intermediate layer of a cellular hydrophilic foam and an outer layer formed from a film having a plurality of openings to allow the passage of air. One disadvantage of this dressing is that none of the layers is a barrier to liquid water and hence to bacteria.

European Patent No. 50514 discloses a dressing in which a net of elastomer is used as a wound facing layer.

Accordingly the present invention provides a laminate wound dressing which consists of a low adherency wound facing layer, an absorbent layer and an outer layer laminated together wherein the wound facing layer comprises a conformable net of elastomer, the intermediate absorbent layer comprises a conformable open cell foam of hydrophilic polymer and the outer layer comprises a conformable microporous film which is a barrier to bacteria and liquid water.

In one aspect the invention provides a low adherency wound dressing which consists of a low adherency wound facing layer, an intermediate absorbent layer and an outer layer as defined above, with a topically effective medicament in the intermediate absorbent layer.

Normally the three layers of the dressing of this invention are attached in a contiguous and coextensive manner; that is the dressing is normally provided in the form of a laminate.

Materials for use in the dressing of the invention and methods of preparing these materials are disclosed in European Patents Nos. 0059048 and 0059049.

Wound dressings of the invention can suitably have a moisture vapour transmission rate of 300 to 5000 grams and preferably 500 to 2000 grams/square meter/24 hours at 37.5°C at 100% to 10% relative humidity difference. It has been found that such moisture vapour transmission rates will allow the wound under the dressing to heal under moist conditions without causing the skin surrounding the wound to macerate.

The conformable net of the elastomer dressing of the invention acts as a low adherency wound facing layer. This layer allows wound exudate to pass to the absorbent layer but prevents the absorbent layer making direct contact with the wound surface.

The net used in this invention is preferably an integral net, that is a net with strands and junctions which have been formed integrally during manufacture.

Preferably the net is sufficiently conformable to allow the wound dressing to conform with the body contours and thereby maintain overall contact with the wound surface to ensure that exudate from the wound is absorbed.

It is also desirable that the net should be sufficiently elastically extensible to adjust to any dimensional changes in the absorbent layer.

Suitable nets will have an elongation at break of 100% to 800% desirably 200% to 750% and preferably 300% to 700% when measured as a 2.5 cm wide strip at a 30 cm/minute strain rate at 20°C.

Normally the net of elastomer is made of a pharmaceutically acceptable wamer insoluble elastomer. Suitable elastomers include polyurethanes, polybutadiene and the like. Preferred polyurethane and polybutadiene elastomers are disclosed in the aforementioned patent applications.

The net of the wound facing layer of the dressing of the invention can have any convenient form depending on the chosen arrangement of strand, junctions and aperture areas and also their shapes and relative size.

Suitable forms of net for the dressings of the invention and the physical characteristics of these nets including preferred numbers and sizes of the net apertures, areas of the voids (apertures), thicknesses and weights of the net are disclosed in the aforementioned patent applications.

The conformable hydrophilic polymer open cell absorbent layer used in dressings of the invention is capable of absorbing wound exudate for example from a burn. It is desirable that the hydrophilic polymer foam layer absorbs the wound exudate rapidly as this enhances the low adherency properties of the absorbent pad. Such rapid absorption prevents undesirable pooling of exudate between the dressing and the wound.

The ability of open cell hydrophilic polymer foam layers to absorb and retain fluids depends to some extent on the size of the foam cells, the porosity of the foam and the thickness of the foam layer. Apt sizes of the foam cells, cell membrane opening areas and thicknesses of the foam are disclosed in the aforementioned patent applications.

The use of such foams of hydrophilic polymer in the absorbent pad of dressings of the invention can allow the wound to be maintained in moist condition even when the exudate produced has been absorbed and removed from the wound surface.

Favoured hydrophilic polymer foams are hydrophilic polyurethane and especially those which are made of crosslinked hydrophilic polyurethane. Preferred foams can be made by reacting a hydrophilic isocyanate terminated polyether prepolymer with water. Favoured hydrophilic polyurethane foams of this type include those known as Hypol foams. Hypol foams can be made from Hypol hydrophilic prepolymers marketed by W. R. Grace and Co.

The conformable film microporous outer layer of the wound dressing of the invention may be used to regulate the moisture loss from the wound area under the dressing and also to act as a barrier to bacteria to delay or prevent bacteria on the outside surface of the dressing penetrating to the wound area.

Suitable conformable microporous films will have a moisture vapour transmission rate of 300 to 5000 grams preferably 500 to 4000 grams/ square metre/24 hrs at 37.5°C at 100% to 10% relative humidity difference. It has been found that such moisture vapour transmission rates of the film allow the wound under the dressing to heal under moist conditions without causing the skin surrounding the wound to macerate.

Suitable conformable microporous films have an average pore diameter of less than 2 microns desirably less than 0.6 microns and preferably less than 0.1 microns. Such microporous films should have an average pore diameter of greater than 0.01 microns.

Suitable conformable microporous films have a thickness of 25 to 400 microns preferably 50 to 300 microns. The conformable microporous film will be made of a polymer. Suitable polymers include plasticised polyvinyl chloride elastomers of polyurethane and ethylene vinyl acetate copolymer elastomers.

A favoured conformable microporous film comprises a microporous plasticised polyvinyl chloride film having an average pore diameter of less than 2 microns, a thickness of 250 to 300 microns and a moisture vapour transmission rate of 3000 to 5000 $g/m^2/24$ hours at 37.5°C at a relative humidity difference of 100% to 10% relative humidity.

The wound dressing of the invention can contain a topically effective medicament. Most suitably the medicament is an antibacterial agent. Preferably the antibacterial agent is a broad spectrum antibacterial agent such as a silver salt for example silver sulphadiazine, an acceptable iodine source such as povidone iodine (also called polyvinyl pyrrolidone iodine or PVP/I), chlorhexidine salts such as the gluconate, acetate, hydrochloride or the like salts or quaternary antibacterial agents such as benzalkonium chloride or the like.

The medicament is preferably located in the foam layer of the dressing.

Preferred amounts of suitable medicaments for incorporation into the foam layer of dressing of the invention are disclosed in the aforementioned patent applications.

The wound dressing of this invention may be in any convenient form of shape or size. In a preferred form the wound dressing is a pad of rectangular shape. In another preferred form the wound dressing can be an elongate strip which may be used as a bandage or may be used to prepare smaller dressings.

It is desirable that the wound dressing of this invention are sterile. The wound dressing of the invention is advantageously provided in bacteria impervious pouches. Such packed forms can be prepared under aseptic conditions or alternatively sterilised after packing by a conventional procedure. A favoured sterilisation procedure is heat sterilisation, for example by steam. Other favoured procedures are ethylene oxide sterilisation or gamma irradiation.

In another aspect the invention provides a process of making a laminate wound dressing of the invention which comprises laminating together a conformable net of elastomer, an intermediate absorbent layer comprising an open cell foam of hydrophilic polymer and an outer layer comprising a conformable microporous film which is a barrier to bacteria and liquid water.

Normally the bringing together of the layers will be a lamination process.

The previously formed individual layers can be formed into a laminate by bonding the layers together in one or more laminating processes. Suitable bonding methods include heat sealing or adhesive bonding providing that the adhesive layer is discontinuous. Such discontinuous adhesive bonding layers will be moisture vapour transmitting and will also allow the passage of wound exudate from the wound facing net layer to the absorbent hydrophilic foam layer when used to bond these layers together.

Preferred discontinuous adhesive bonding layers comprise a pattern of intersecting sets of parallel lines of adhesive for example a diamond pattern which gives adhesive free areas of rectangules, parallelograms and like shapes. Methods of preparing such discontinuous pattern adhesive layers are disclosed in British Patent No. 819,635. Such patterns can be produced by spraying-on the adhesive. Favoured adhesive bonding compositions include acrylate ester copolymers and polyvinyl ethyl ethers.

A preferred bonding method for forming the film/foam/net laminate of the invention is heat sealing. The net and film layers can be heat sealed to the foam layer by heat and pressure in a conventional manner in one or more laminating processes. An apt heat sealing process comprises passing the net or film layer in contact with the foam layer through the nip of a heated metal roller and rubber roller under low pressure. To ensure that the net or film is in a heat softened state it is desirable that the net or film layer is adjacent to the heated metal roller. Thus by this process the laminate can be formed in two consecutive operations in which for example the film layer is laminated to the foam layer in a first pass through the laminating rollers and the net layer to the opposed face of the foam in a second pass through the rollers. Alternatively, the laminate can be formed in one operation by passing the layers through the nips of two sets of laminating rollers.

When the net has been formed on an embossed film casting sheet, it is preferred that the net is supported on its embossed film casting sheet during the heat lamination process. It has been found with this arrangement that the supported net has less tendency to be compressed and 'flattened' into the surface of the foam by heat and pressure of laminating process thus ensuring that the net is a discrete layer on the foam surface.

In a continuous process the wound dressing can be made in the form of a continuous strip which is then cut up into suitable sized dressings.

Processes for forming the materials used in the dressings of the invention including the preferred hydrophilic polyurethane foam layers, the preferred polyurethane elastomer net layers, the preferred acrylate ester copolymer and polyvinyl ethyl ether adhesive bonding compositions and laminates of these materials are disclosed in the aforementioned patent applications.

Suitable microporous films for the outer layer of a wound dressing of the invention can be made by the method disclosed in British Patent No. 884,232.

The invention will now be illustrayed by the following examples.

Preparation of net

An integral diamond pattern polyurethane net of which 4 apertures/cm was prepared in the same manner as in Example 22 of British Patent Application No. 8204132 (GB—A—2 093 702) using a melt embossed polypropylene sheet (polypropylene containing 40% by weight chalk filler reference PXC 4999 available from ICI Plastics Limited) instead of a high density polyethylene sheet.

Preparation of the absorbent layer

Using the two component dispensing Vari-o-mix (supplied by Prodef Engineering Limited) a foaming mixture was formed by mixing Hypol FHP 2002 and Brig 72 (1% aqueous solution) in the ratio of 1:2. The foaming mixture was put into the coating head by means of an output nozzle in the form of an 15 cm wide 'fishtail die' and coated onto a silicone coated release paper (Stearalese No. 46 available from Sterling Coated Papers Limited) by means of a knife over roller coating head set at a gap of 1 mm. The cast foam was dried by passage through an air circulating oven at a temperature of 50°C for 5 minutes. The cast hydrophilic polyurethane foam had a thickness of 2 mm.

Preparation of the film layer

A microporous plasticised polyvinyl chloride film (275 microns thick) having an average pore diameter of less than 2 microns was made by the method disclosed in British Patent No. 884,232. The film was then coated with discontinuous pattern of a polyvinyl ethyl ether pressure sensitive adhesive solution (adhesive composition A of British Patent No. 1,280,631) by the method given in British Patent No. 819,635 and passed into a heated oven to give a dry weight per unit area of 30 g/m². The discontinuous adhesive coating had diamond pattern of adhesive free areas between intersecting parallel lines of adhesive (4 per cm). The adhesive coated film was laminated to a silicone coated release protector layer (Steralease No. 15 available from Sterling Coated Papers Limited).

Preparation of low adherency wound dressings

The polyurethane net on its embossed casting sheet was heat laminated to the hydrophilic polyurethane foam on its silicone coated release casting paper by passing the layers between the nip of a silicone rubber roller and a steel roller heated by circulating oil to a temperature of 135°C. The embossed sheet carrying the polyurethane net was fed against the heated steel roller to ensure that the net was in a heat softened condition prior to its lamination to the foam.

The silicone coated release paper was then removed from the foam layer of net/foam laminate and the adhesive coated microporous film (with protector removed) was laminated to the foam surface by a similar laminating process in which the steel roller was maintained at room temperature.

The embossed carrier sheet was then removed from the net surface to give a three layer laminate strip and the strip cut into dressings.

Demonstration of effectiveness
Absorbency testing

A dressing formed as described in the Example cut to a size of 5.2 cm×5.2 cm (approximately) was placed under light pressure with the net-carrying surface of the foam in contact with horse serum. The serum was available through an orifice 1 cm in diameter at zero hydrostatic pressure. The penetration of the serum was followed by observation and by weighing the dressing before and at intervals during the absorption process. Initially the rate of absorption was slow but increased rapidly so that, after 30 minutes from the start of the experiment, the pad was observed to be saturated and contained 8 g of serum, as measured by the weight difference between the start and end of the experiment.

The experiment showed that the absorption capacity of the foam was not restricted by the presence of a net on one surface and a film on the other.

Demonstration of effectiveness
Moisture vapour permeability (MVP) determination

Discs of the dressing material that is a laminate of net/foam/adhesive/backing layer, to be tested are clamped over Payne Permeability Cups (flanged metal cups) using sealing rings and screw clamps. The exposed surface of the test sample is 10 cm$^2$. Each cup contains approximately 10 ml of distilled water.

After weighing the cups are placed in a fan assisted electric oven maintained at 37.5°. The relative humidity within the oven is maintained at approximately 10% by placing 1 kg of anhydrous 3—8 mesh calcium chloride on the floor of the oven.

The cups are moved after 24 hours, allowed to cool for 20 minutes and reweighed. The MVP of the test material is calculated from the weight loss and expressed in units of grams of weight per square meter per 24 hours, at 37.5°C at 100—10% relative humidity difference.

The results were as follows:

| 5 samples of | Moisture vapour permeability (g/m$^2$/24 hrs) |
|---|---|
| Dressing material (ex Example) | 1616, 1612, 1866, 1720, 1740 |

**Claims**

1. A laminate wound dressing which consists of a low adherency wound facing layer, an absorbent layer and an outer layer laminated together wherein the wound facing layer comprises a conformable net of elastomer, the intermediate absorbent layer comprises a conformable open cell foam of hydrophilic polymer and the outer layer comprises a conformable microporous film which is a barrier to bacteria and liquid water.

2. A wound dressing according to claim 1 in which a topically effective medicament is present in the intermediate absorbent layer.

3. A wound dressing according to claim 1, wherein the outer layer has a moisture vapour transmission rate of 500 to 4000 grams/square meter/24 hours at 37.5°C at 100% to 10% relative humidity difference.

4. A wound dressing according to claim 1 wherein the microporous film outer layer has a mean pore diameter of 0.01 to 2 microns.

5. A wound dressing according to claim 1, wherein the microporous film outer layer has a thickness of 50 to 300 microns.

6. A wound dressing according to claim 1, wherein the outer layer is a microporous plasticised polyvinyl chloride film having an average pore diameter of 0.01 to 2 microns, a thickness of 250 to 300 microns and a moisture vapour transmission rate of 3000 to 5000 g/m$^2$/24 hours at 37.5°C at a relative humidity difference of 100% to 10% relative humidity.

7. A wound dressing according to claim 6, wherein the average pore diameter is 0.01 to 0.1 microns.

8. A wound dressing according to claim 6, wherein the intermediate absorbent layer is a cross-linked hydrophilic polyurethane foam made by reacting a hydrophilic isocyanate terminate polyether prepolymer with water, and optionally contains a topically effective medicament.

9. A wound dressing according to claim 6, wherein the wound facing layer is an integral net.

10. A process of making a laminate wound dressing according to claim 1, which comprises laminating together a conformable net of elastometer, an intermediate absorbent layer comprising an open cell foam of hydrophilic polymer and an outer layer comprising a conformable microporous film which is a barrier to bacteria and liquid water.

11. A process according to claim 10 in which the layers are laminated together by means of heat sealing.

## Patentansprüche

1. Laminierter Wundverband, der aus einer der Wunde zugekehrten Schicht mit geringer Haftfestigkeit, einer absorbierenden Schicht und einer äußeren Schicht besteht, die zusammenlaminiert sind, wobei die der Wunde zugekehrte Schicht ein sich anpassendes Elastomernetz, die absorbierende Zwischenschicht einen sich anpassenden offenzelligen Schaum aus hydrophilem Polymer und die äußere Schicht einen sich anpassenden mikroporösen Film aufweist, der eine Sperre für Bakterien und flüssiges Wasser darstellt.

2. Wundverband nach Anspruch 1, bei welchem ein äußerlich wirksames Medikament in der absorbierenden Zwischenschicht vorhanden ist.

3. Wundverband nach Anspruch 1, bei welchem die äußere Schicht eine Durchlässigkeit für Wasserdampf von 500—4000 g/m²/24h bei 37,5°C bei 100—10% rel. Feuchtigkeitsdifferenz aufweist.

4. Wundverband nach Anspruch 1, bei welchem die aus einem mikroporösen Film bestehende äußere Schicht einen mittleren Porendurchmesser von 0,01—2 Mikrometer aufweist.

5. Wundverband nach Anspruch 1, bei welchem die aus einem mikroporösem Film bestehende äußere Schicht eine Stärke von 50—300 Mikrometer aufweist.

6. Wundverband nach Anspruch 1, bei welchem die äußere Schicht aus einem mikropösen Film aus weichgestelltem PVC mit einem durchschnittlichen Porendurchmesser von 0,01—2 Mikrometer, einer Dicke von 250—300 Mikrometer und einer Durchlässigkeit für Wasserdampf von 3000—5000 g/m²/24h bei 37,5°C bei einer rel. Feuchtigkeitsdifferenz von 100%—10% rel. Feuchte besteht.

7. Wundverband nach Anspruch 6, bei welchem der durchschnittliche Porendurchmesser 0,01—0,1 Mikrometer beträgt.

8. Wundverband nach Anspruch 6, bei welchem die absorbierende Zwischenschicht ein vernetzter hydrophiler Polyurethan-Schaum ist, der durch Umsetzen eines hydrophilen, mit Isocyanatendgruppen versehen Polyätherprepolymers mit Wasser hergestellt worden ist, und wahlweise ein äußerlich wirksames Medikament enthält.

9. Wundverband nach Anspruch 6, bei welchem die der Wunde zugekehrte Schicht als integrales Netz ausgebildet ist.

10. Verfahren zum Herstellen eines laminierten Wundverbandes nach Anspruch 1, bei welchem ein sich anpassendes Elastomernetz, eine absorbierende Zwischenschicht mit einem offenzelligen Schaum aus hydrophilem Polymer und eine äußere Schicht mit einem sich anpassenden mikroporösen Film zusammenlaminiert werden, der eine Sperre für Bakterien und flüssiges Wasser darstellt.

11. Verfahren nach Anspruch 10, bei welchem die Schichten mittels Heißsiegeln zusammenlaminiert werden.

## Revendications

1. Pansement feuilleté pour plaie qui comprend une couche faisant face à la plaie de faible adhérence, une couche absorbante et une couche extérieure feuilletées ensemble, dans lequel la couche faisant face à la plaie comprend un filet adaptable d'élastomère, la couche absorbante intermédiaire comprend une mousse adaptable à cellules ouvertes de polymère hydrophile et la couche extérieure comprend un film microporeux adaptable qui est une barrière pour les bactéries et l'eau liquide.

2. Pansement pour plaie suivant la revendication 1, dans lequel un médicament efficace localement est présent dans la couche absorbante intermédiaire.

3. Pansement pour plaie suivant la revendication 1, dans lequel la couche extérieure a un taux de transmission de vapeur d'eau de 500 à 4000 $g.m^{-2}.24h^{-1}$ à 37,5°C pour une difference d'humidité relative de 100% à 10%.

4. Pansement pour plaie suivant la revendication 1, dans lequel la couche extérieure de film microporeux a un diamètre de pore moyen de 0,01 à 2 microns.

5. Pansement pour plaie suivant la revendication 1, dans lequel la couche extérieure de film microporeux a une épaisseur de 50 à 300 microns.

6. Pansement pour plaie suivant la revendication 1, dans lequel la couche extérieure est un film microporeux de chlorure de polyvinyle plastifié ayant un diamètre de pore moyen de 0.01 à 2 microns, une épaisseur de 250 à 300 microns et un taux de transmission de vapeur d'eau de 3000 à 5000 $g.m^{-2}.24\ h^{-1}$ à 37,5°C pour une difference d'humidité relative de 100% à 10%.

7. Pansement pour plaie suivant la revendication 6, dans lequel le diamètre de pore moyen est de 0,01 à 0,1 micron.

8. Pansement pour plaie suivant la revendication 6, dans lequel la couche absorbante intermédiaire est une mousse de polyuréthanne hydrophile réticulé, obtenue par réaction avec de l'eau d'un prépolymère hydrophile de polyéther à terminaison isocyanate, et contient éventuellement un médicament efficace localement.

9. Pansement pour plaie suivant la revendication 6, dans lequel la couche faisant face à la plaie est un filet intégral.

10. Procédé de fabrication d'un pansement fouilleté pour plaie suivant la revendication 1, qui comprend le feuilletage d'un filet adaptable d'élastomère, d'une couche absorbante intermédiaire comprenant une mousse à cellules ouvertes de polymère hydrophile et d'une couche extérieure comprenant un film microporeux adaptable qui est une barrière pour les bactéries et l'eau liquide.

11. Procédé suivant la revendication 10, dans lequel les couches sont feuilletées ensemble par thermosoudure.